(19) Europäisches Patentamt

European Patent Office

Office européen des brevets

(11) Veröffentlichungsnummer: **0 200 157 B1**

(12) **EUROPÄISCHE PATENTSCHRIFT**

(45) Veröffentlichungstag der Patentschrift: **08.07.92**

(21) Anmeldenummer: **86105648.9**

(22) Anmeldetag: **24.04.86**

(51) Int. Cl.⁵: **A61K 33/40**

(54) Verwendung einer wässrigen stabilisierten Chloritmatrixlösungzur Herstellung von Arzneimitteln zur intravenösen Applikation bei infektiösenZuständen.

(30) Priorität: **02.05.85 DE 3515749**

(43) Veröffentlichungstag der Anmeldung:
**05.11.86 Patentblatt 86/45**

(45) Bekanntmachung des Hinweises auf die
Patenterteilung:
**08.07.92 Patentblatt 92/28**

(84) Benannte Vertragsstaaten:
**AT BE CH DE FR GB IT LI LU NL SE**

(56) Entgegenhaltungen:
**EP-A- 0 093 875**
**US-A- 4 296 102**

**NONLISTED DRUGS, Band 36, Nr. 3, März
1984, Seite 51, Chatham,NY, US; "Oxoferin"**

**CHEMICAL ABSTRACTS, 74, 1971, Seite 375,
Nr. 139179m, Columbus, Ohio, US; A. BILLIAU
et al.: "Effect of chlorite-oxidized oxyamylose on influenza virus infection in mice"**

(73) Patentinhaber: **OXO Chemie GmbH
Im Neuenheimer Feld 517
W-6900 Heidelberg(DE)**

(72) Erfinder: **Kühne, Friedrich W., Dr.
Bergstrasse 72
W-6900 Heidelberg(DE)**
Erfinder: **Elstner, Erich F., Prof. Dr.
Wildmoos-Strasse 18
W-8038 Gröbenzell(DE)**
Erfinder: **Kühne, Hans-Heinrich, Dr.
Holtenauer Strasse 83
W-2300 Kiel(DE)**

(74) Vertreter: **Grussdorf, Jürgen, Dr. et al
Patentanwälte Zellentin & Partner Rubensstrasse 30
W-6700 Ludwigshafen(DE)**

## Beschreibung

Die Erfindung betrifft die Verwendung einer wässrigen Lösung einer stabilisierten Chloritmatrix zur Herstellung von Arzneimitteln zur intravenösen Applikation bei infektiösen Zuständen, hervorgerufen durch Parasiten, Pilze, Bakterien, Vieren und/oder Mykoplasmen.

Stabilisierte Chloritmatrices sind aus der US-A-4,296,103 und EP-A 0093875 zur oralen bzw. parenteralen Applikation bekannt. Wegen der Toxicität der Lösungen wurde angenommen, daß eine intravenöse Applikation nicht möglich ist.

Es wurde gefunden, daß stabilisierte Chloritmatrices, die mit Hilfe von Bioaktivatoren zu elektronenaffinen Verbindungen aktiviert werden können, unabhängig von ihrem Sauerstoffgehalt, die "OXIDATIVE BURST" Antwort von Phagozyten amplifizieren. Aus der Literatur ('Enhancement of Phagocytosis-Associated Metabolism as a Manifestation of Macrophage Activation' by Richard B. Johnston, Dr. in Lymphokines, 3:33-56, 1981, Academic Press) ist bekannt, dass eine enge Korrelation zwischen dem Ausmass der oxidativen Antwort auf die Phagozytose und der Fähigkeit, intrazellulär Mikroorganismen abzutöten, besteht. In vitro stellt die mit dem 'OXIDATIVE BURST' einhergehende Chemiluminsezenz ein quantitatives Mass für die Phagozyten-Stimulierung dar. Messungen, bei denen menschliche Granulozyten in Vollblut mit unspezifisch und spezifisch opsonierten humanpathogenen Bakterien inkubiert wurden, zeigten, daß durch eine Zugabe von stabilisierter Chloritmatrixlösung die Chemilumineszenz auf das 1.7 - 17-fache des Kontrollwertes zunehmen kann, sofern Häm-haltige Bioaktivatoren in dem Medium frei gelöst verfügbar sind.

Die in vitro gemessene Phagozyten-Stimulierung wurde durch die in vivo registrierte Phagozyten-Aktivierung (Phagokinese) bestätigt. Bei der C57 B1/6 Maus führte eine durch Verabreichung (einmalige i.p. Gaben von 0,2 - 0,5 ml Chloritmatrix per kg Körpergewicht eine Stunde nach i.v. Gabe von $4,5 \times 10^5$ listeria monocytogenes Bakterien entsprechend einer infektiös letalen Dosis für 80 % der Tiere ($ILD_{80}$)) einer Lösung von stabilisierter Chloritmatrix induzierte Phagokinese zu signifikant erhöhter listeria monocytogenes Clearance in der Milz bzw. zum Überleben aller Tiere.

Nach intravenöser Infektion von BalbC Mäusen mit einer $ILD_{75}$ von candida albicans, welche gegenüber menschlichen nichtaktivierten Blutphagozyten eine bemerkenswerte Resistenz aufweist, konnte die Überlebensrate durch eine einmalige, eine Stunde nach der Infektion erfolgte intravenöse Applikation von 0,2 ml Chloritmatrix-Lösung pro kg Körpergewicht gegenüber den Kontrolltieren, welche 0,9 % NaCl i.v. erhielten, auf das Doppelte erhöht werden (15/30 gegenüber 7/30).

In vitro Untersuchungen haben weiterhin gezeigt, dass stabilisierte Chloritmatrix-Häm-Komplexe nicht nur eine Degranulation von Neutrophilen innerhalb von Minuten einleiten, sondern auch direkt bakterizid und fungizid wirken. Die bakterizide Wirkung erstreckt sich sowohl auf aerobe wie auf anaerobe Mikroorganismen.

Erfindungsgemäß wird daher die Verwendung einer wässrigen Lösung einer stabilisierten Chloritmatrix zur Herstellung von Arzneimitteln zur intravenösen Applikation bei infektiösen Zuständen, hervorgerufen durch Parasiten, Pilze, Bakterien und/oder Mykoplasmen, vorgeschlagen.

## Patentansprüche

1. Verwendung einer wässrigen Lösung einer stabilisierten Chloritmatrix zur Herstellung von Arzneimitteln zur intravenösen Applikation bei infektiösen Zuständen, hervorgerufen durch Parasiten, Pilze, Bakterien und/oder Mykoplasmen.

## Claims

1. Use of an aqueous solution of a stabilised chlorite matrix for the preparation of medicaments for intravenous administration in the case of infectious conditions brought about by parasites, fungi, bacteria and/or Mycoplasmae.

## Revendications

1. Utilisation d'une solution aqueuse d'une matrice de chlorite stabilisée pour la préparation de médicaments destinés à être appliqués par voie intraveineuse dans le cas d'états infectieux, engendrés par des parasites, des champignons, des bactéries et/ou des mycoplasmes.